(19) Europäisches Patentamt
European Patent Office
Office européen des brevets

(11) EP 0 638 104 B1

(12) **FASCICULE DE BREVET EUROPEEN**

(45) Date de publication et mention
de la délivrance du brevet:
**22.07.1998   Bulletin 1998/30**

(21) Numéro de dépôt: **93911825.3**

(22) Date de dépôt: **27.04.1993**

(51) Int Cl.6: **C08H 1/00**, C08B 37/00,
A61K 7/48

(86) Numéro de dépôt international:
**PCT/FR93/00406**

(87) Numéro de publication internationale:
**WO 93/22370 (11.11.1993 Gazette 1993/27)**

(54) **DERIVES D'ACIDE UNDECYLENIQUE AVEC DES MACROMOLECULES HYDROPHILES ET UTILISATIONS EN COSMETIQUE OU EN PHARMACIE**

UNDECYLENSÄUREDERIVATE MIT HYDROPHILEN MAKROMOLEKÜLEN UND DEREN KOSMETISCHE ODER PHARMAZEUTISCHE VERWENDUNGEN

UNDECYLENIC ACID DERIVATIVES WITH HYDROPHILIC MOLECULES AND UTILIZATIONS IN COSMETICS OR IN PHARMACY

(84) Etats contractants désignés:
**BE DE ES FR IT NL**

(30) Priorité: **27.04.1992  FR 9205181**

(43) Date de publication de la demande:
**15.02.1995   Bulletin 1995/07**

(73) Titulaire: **COLETICA**
**69007 Lyon (FR)**

(72) Inventeurs:
• **PERRIER, Eric, Jean-Luc**
  **F-38200 Vienne (FR)**
• **ANTONI, Danielle 23, domaine des Essarts**
  **F-69390 Vernaison (FR)**
• **HUC, Alain, Roger**
  **F-69110 Sainte-Foy-les-Lyon (FR)**

(74) Mandataire: **Portal, Gérard**
**Cabinet Beau de Loménie**
**158, rue de l'Université**
**75340 Paris Cédex 07 (FR)**

(56) Documents cités:
**EP-A- 0 417 619          WO-A-92/13006**
**FR-A- 2 009 161          FR-A- 2 122 284**
**US-A- 4 234 475**

• **COSMETICS & TOILETRIES vol. 104, Septembre 1989, pages 87 - 95 D. B. BRAUN 'Developments with lipoamino acids and their salts'**
• **HIGH POLYMERS VOL. V, CELLULOSE AND CELLULOSE DERIVATIVES , PART II, ED. 1954, ED. BY OTT & SPURLIN, SECOND EDITION 1954, pages 763 - 824 C. J. MALM & G. D. HIATT 'Organic esters'**
• **CHEMICAL ABSTRACTS, vol. 82, no. 16, 21 Avril 1975, Columbus, Ohio, US; abstract no. 103149c, 'Undecylenoyl amino acids for the treatment of skin disorders' page 358 ;**

## Description

La présente invention conceme essentiellement l'utilisation de dérivés d'acide undécylénique avec des macromolécules hydrophiles comme agents anti-microbiens, anti-bactériens ou anti-fongiques, en cosmétique ou pharmacie. Dans le cadre de ces utilisations, ces compositions peuvent présenter une activité anti-sudorale, anti-pelliculaire, anti-acnéique. Ces dérivés de l'acide undécylénique avec des macromolécules hydrophiles peuvent être avantageusement utilisés comme émulsionnant et comme agent conservateur principal ou auxiliaire de compositions cosmétiques ou pharmaceutiques.

On sait que l'acide undécylénique est un acide gras dont la chaîne comporte 11 atomes de carbone et une insaturation entre le 10e et le 11e atome. Cet acide gras est obtenu par vapo-craquage de l'huile de ricin, et, bien que rarement rencontré dans la nature, il se trouve à l'état naturel dans les larmes et la sueur. Il est insoluble dans l'eau et présente une odeur très forte qui a été utilisée pour masquer d'autres odeurs agro-industrielles ou industrielles désagréables.

La principale caractéristique de l'acide undécylénique réside dans le fait que, comme les acides gras saturés à chaîne courte ayant moins de 8 atomes de carbone, il présente une activité anti-fongique et anti-bactérienne très forte.

Le spectre d'action anti-microbien de l'acide undécylénique sur les bactéries Gram +, Gram - sur les levures et sur les champignons est connu, mais la législation cosmétique française et européenne ne permet pas son utilisation en tant que conservateur à une concentration supérieure à 0,2 % en poids, ce qui limite son emploi dans le domaine cosmétique.

Il a également été proposé l'emploi des sels de l'acide undécylénique tels que les sels de calcium, de zinc, de sodium, de potassium. Ils présentent tous une activité anti-fongique et anti-bactérienne proche de celle obtenue avec l'acide undécylénique, particulièrement lorsque le pH d'utilisation est compris entre 5 et 6,5. Cependant, les sels présentent le même problème d'odeur que l'acide correspondant et la législation ne les autorise pas à une concentration supérieure à 0,2 % en poids lorsqu'ils sont utilisés en tant que conservateur.

Il a été décrit dans le document US-A-4 234 475, un procédé d'association entre une protéine et un acide gras. Le procédé décrit implique le maintien à haute température de la protéine et de l'acide gras, ce qui entraîne la dégradation de ces deux substances. En outre, le procédé décrit ne crée pas de liaison covalente entre l'acide et la protéine. En outre, la réaction peut avoir lieu dans un solvant inerte c'est-à-dire pour un homme de l'art dans un solvant organique.

Dans le cadre de l'invention, on recherche des dérivés de l'acide undécylénique avec une macromolécule organique hydrophile, préparés à la température ambiante pour éviter toute dégradation de ces macromolécules et d'autre part en évitant l'emploi de solvant, en préférant ainsi une réaction en milieu aqueux.

Avec ces impératifs, les inventeurs ont découvert de nouveaux dérivés de l'acide undécylénique présentant des propriétés inattendues, comme cela ressort de la description suivante et des revendications.

La présente invention a donc pour but de foumir des dérivés de l'acide undécylénique, très faiblement odorants, présentant des propriétés antifongiques et anti-bactériennes encore très importantes pour être mises à profit dans de très nombreuses formulations cosmétiques.

La présente invention a encore pour but de foumir des dérivés de l'acide undécylénique, présentant de nouvelles propriétés intéressantes. Dans ce cadre, il a été découvert des nouveaux dérivés de l'acide undécylénique qui présentent de nouvelles propriétés anti-sudorale, anti-pelliculaire, anti-acnéique, un fort pouvoir hydratant ou émulsionnant ainsi qu'une toxicité moindre permettant un emploi fréquent dans le domaine cosmétique ou pharmaceutique.

La présente invention a encore pour but de foumir de nouvelles compositions cosmétiques ou pharmaceutiques contenant de tels nouveaux dérivés de l'acide undécylénique.

La présente invention a enfin pour but de foumir l'utilisation de dérivés de l'acide undécylénique comme agents actifs pour la fabrication d'une composition cosmétique ou pharmaceutique, à activité anti-microbienne, anti-bactérienne ou anti-fongique.

Tous ces buts sont atteints simultanément selon la présente invention de manière simple et utilisable à l'échelle industrielle cosmétique ou pharmaceutique.

Ainsi, selon un premier aspect, la présente invention concerne l'utilisation d'un dérivé de l'acide undécylénique sous forme du produit de réaction en milieu aqueux, à température ambiante, de l'acide undécylénique avec au moins une macromolécule organique hydrophile possédant des groupements alcool primaire et/ou amine primaire, ayant une masse moléculaire supérieure à 2 000 Daltons, comme agent anti-microbien, ou anti-bactérien, ou anti-fongique.

Dans la présente description et les revendications, le terme "macromolécule organique hydrophile" signifie toute macromolécule organique hydrophile ayant une masse moléculaire supérieure à 2 000 g/mol, c'est-à-dire 2 000 Daltons, de préférence une masse moléculaire supérieure à 5 000 Daltons.

Il est à noter que de telles macromolécules organiques hydrophiles comprennent habituellement des protéines, des polysaccharides ou des acides nucléiques.

Par contre, ne sont pas considérées comme des macromolécules en raison de leur masse moléculaire inférieure à 2 000 Daltons, les aminoacides qui présentent une masse moléculaire moyenne de 130 Daltons, les peptides, par exemple à cinq acides aminées ayant une masse moléculaire moyenne de 650 Daltons, un monosaccharide ayant une masse moléculaire moyenne de 180 Daltons, un oligosaccharide ayant une masse moléculaire moyenne de 900 Daltons.

Selon un mode de réalisation avantageux, le dérivé précité d'acide undécylénique comprend une proportion relative d'acide undécylénique par rapport à la macromolécule organique hydrophile allant de 10 à 75 % en poids, de préférence comprise entre 50 et 75 % en poids.

Selon un mode de réalisation avantageux, ce produit de réaction a en outre subi une étape de lyophilisation.

Selon un mode de réalisation particulier, l'acide undécylénique est mis à réagir sous une forme réactive bien connue à l'homme de l'art, par exemple sous forme d'halogénure tel que chlorure, fluorure ou à la rigueur iodure, ou sous forme d'anhydride. La forme préférée est un chlorure ou un anhydride de l'acide undécylénique.

Selon un mode de réalisation particulièrement avantageux de l'invention, comme macromolécule organique hydrophile mise à réagir avec l'acide undécylénique sous forme réactive, on utilise une macromolécule organique hydrophile de masse moléculaire supérieure à 5 000 Daltons (D), de préférence comprise entre 5 000 D et 1 000 000 D, encore de préférence comprise entre 50 000 D et 300 000 D. De telles macromolécules peuvent être d'origine naturelle ou synthétique. Cette macromolécule organique d'origine naturelle peut être d'origine animale, végétale, ou marine. Lorsqu'elle est d'origine synthétique, elle peut être obtenue par fermentation.

L'origine de ces macromolécules peut être animale et, dans ce cas, les protéines suivantes sont particulièrement préférées : kératine, soie, collagène, réticuline, élastine, lactalbumine, lactoglobulinc, caséine, ovalbumine ; ainsi que les polysaccharides suivants: acide hyaluronique, chondroïtine-4-sulfate, chondroïtine-6-sulfate, dermatane sulfate, héparane sulfate et kératane sulfate ainsi que l'héparine et ses dérivés ou ses fractions de masse moléculaire supérieure à 5 000 D.

Comme macromolécule organique hydrophile comprenant des groupements amine primaire, on peut citer les acides nucléiques tels que l'ADN, l'ARN ou leurs fragments.

Lorsque l'origine de ces macromolécules est végétale, on préfère les protéines suivantes: protéines de blé, protéines de germes de blé, de maïs, d'avoine, de soja, d'amande ainsi que les polysaccharides suivants: amylose, amylopectine, glucomannanes, galactomannanes, fructosanes, gommes, celluloses et dérivés.

Lorsque l'origine de ces macromolécules est marine, on préfère les protéines suivantes : collagène, protéines de poissons, protéines de mollusques, protéines de crustacés, protéines d'algues, ainsi que les polysaccharides suivants: chondroïtine sulfate, chitosan, alginates, agar , carraghénane.

Les macromolécules organiques hydrophiles selon l'invention peuvent également être obtenues par fermentation et dans ce cas on préfère utiliser les polysaccharides suivants : xanthane, gellane, curdlane, dextrane.

Des dérivés de l'acide undécylénique particulièrement préférés sont les suivants :

- produit de réaction d'acide undécylénique - protéine de blé de haut poids moléculaire, c'est-à-dire de poids moléculaire supérieur ou égal à environ 5 000 D ;
- produit de réaction d'acide undécylénique - protéines d'algues spirulines ;
- produit de réaction d'acide undécylénique - collagène extrait de peau de poissons;
- produit de réaction d'acide undécylénique - xanthane;
- produit de réaction d'acide undécylénique - ADN de haut poids moléculaire, c'est-à-dire de poids moléculaire supérieur ou égal à environ 5 000 D ;
- produit de réaction d'un mélange acide undécylénique - acide gras, en particulier l'acide laurique avec l'ADN de haut poids moléculaire ci-dessus ;
- produit de réaction d'acide undécylénique - protéines de soie de haut poids moléculaire ;
- produit de réaction d'acide undécylénique - kératine ;
- produit de réaction d'acide undécylénique - protéine d'au moins 5000D extraite d'algues roses.

Selon un mode de réalisation particulièrement avantageux de l'invention, le produit de réaction précité est neutralisé par une base organique ou minérale. Cette neutralisation peut être partielle ou complète. Comme base organique, on peut utiliser avantageusement une amine ou une alkylamine ayant de 1 à 6 atomes de carbone. Comme amine préférée, on utilise la monoéthanolamine, la diéthanolamine, la triéthanolamine. Comme base minérale, on utilise de préférence NaOH, KOH ou une base métallique notamment sous forme d'hydroxyde ou de carbonate tel qu'hydroxyde de zinc, hydroxyde carbonate de zinc et hydroxyde d'aluminium. Ces dérivés ainsi neutralisés par des bases organiques ou minérales permettent d'apporter des propriétés intéressantes au produit final, de préférence permettent d'apporter des propriétés anti-sudorales, ainsi que des propriétés anti-bactériennes améliorées.

Selon une autre variante de réalisation particulière, l'acide undécylénique peut être mis à réagir en mélange avec un autre acide gras saturé ou insaturé, en particulier ayant de 8 à 28 atomes de carbone. Des exemples particulièrement avantageux de l'acide gras sont l'acide stéarique, l'acide laurique, l'acide palmitique, l'acide myristique, l'acide caprylique.

Les proportions relatives d'acide undécylénique par rapport à la macromolécule peuvent varier dans de larges limites, mais une proportion relative préférée d'acide undécylénique par rapport à la macromolécule hydrophile est comprise entre au moins 10 et 75 % en poids et de préférence entre 15 et 75 % en poids. Ainsi, la proportion relative d'acide undécylénique est très élevée par rapport à la macromolécule, cette proportion ne pouvant pas être obtenue si seules les liaisons covalentes sont présentes entre les deux constituants. Une telle proportion aussi élevée est obtenue de manière critique par l'emploi d'une étape de lyophilisation. Avant de réaliser cette étape, il est également préféré d'amener le pH du milieu réactionnel à une valeur basique proche de la neutralité. Compte tenu du fait que, dans le cas de l'invention, on peut préparer selon des variantes de réalisation avantageuses des produits hydratants et émulsionnants, destinés à être utilisés pour la fabrication d'une composition cosmétique ou pharmaceutique, on préfère amener le pH à une valeur comprise entre 5,5 et 7,5.

On a observé que, pour obtenir une activité hydratante significative du produit de réaction acide undécylénique - macromolécule, la proportion relative en acide undécylénique est avantageusement comprise entre 10 et 75 % en poids et de préférence entre 50 et 75 % en poids. D'autre part, pour obtenir une activité émulsionnante significative du produit de réaction acide undécylénique - macromolécule, il est préféré un rapport relatif d'acide undécylénique/macro-molécule compris entre 50 % et 75 % en poids.

Dans le cadre de l'utilisation du dérivé de l'acide undécylénique - macromolécule comme agent anti-pelliculaire, la proportion relative en acide undécylénique peut être avantageusement comprise entre 50 et 75 % en poids.

Lors de l'utilisation du dérivé de l'acide undécylénique - macromolécule comme agent anti-acnéique, la proportion relative en acide undécylénique est avantageusement comprise entre 50 et 75 % en poids.

Lors de l'utilisation du dérivé de l'acide undécylénique - macromolécule comme agent déodorant et/ou agent anti-sudoral, la proportion relative en acide undécylénique dans le produit de réaction acide undécylénique - macromolécule est avantageusement comprise entre 50 et 75 % en poids.

La présente invention concerne, selon un deuxième aspect, l'utilisation du dérivé acide undécylénique - macromolécule organique hydrophile précité comme agent actif, en particulier anti-bactérien ou anti-fongique, pour la fabrication d'une composition cosmétique ou pharmaceutique. Dans cette utilisation, toute proportion du dérivé acide undécylénique - macromolécule peut être utilisée. Des proportions en poids particulièrement préférées sont comprises entre 0,1 et 5 % en poids par rapport au poids total de la composition.

Dans le cadre de cette utilisation, le dérivé acide undécylénique - macromolécule organique hydrophile peut constituer un agent émulsionnant.

Egalement, le dérivé acide undécylénique - macromolécule organique hydrophile peut constituer un agent hydratant. Dans l'utilisation comme agent émulsionnant ou comme agent hydratant, des proportions particulièrement préférées sont comprises entre 0,1 et 5 % en poids par rapport au poids total de la composition.

Le dérivé acide undécylénique - macromolécule organique hydrophile peut également être utilisé comme agent anti-pelliculaire et, dans ce cadre, une proportion particulièrement préférée est comprise entre 0,1 et 5 % en poids. Les dérivés particulièrement préférés ayant une activité anti-pelliculaire sont les dérivés de l'acide undécylénique avec les protéines de haut poids moléculaire de la soie, du collagène, de la kératine, des protéines d'algues roses sous forme d'extrait classique disponible dans le commerce.

Le dérivé acide undécylénique - macromolécule organique hydrophile peut également être utilisé comme agent anti-acnéique pour la fabrication de compositions cosmétiques ou pharmaceutiques. Dans ce cadre, la proportion en poids est de préférence comprise entre 0,1 et 5 % en poids.

Le dérivé de l'acide undécylénique - macromolécule organique hydrophile selon l'invention peut également être utilisé comme agent déodorant et anti-sudoral et dans ce cadre de la proportion relative en poids est avantageusement comprise entre 0,1 % et 5 % par rapport au poids de la composition totale.

Le dérivé selon l'invention peut également être utilisé comme agent conservateur principal ou auxiliaire, en raison de ses propriétés anti-bactériennes ou anti-fongiques, d'une composition cosmétique ou pharmaceutique. Dans ce cas, la proportion sera également avantageusement comprise entre 0,1 et 5 % en poids, de préférence entre environ 1 et environ 3 % en poids par rapport au poids total de la composition.

L'invention conceme encore l'utilisation d'un dérivé de l'acide undécylénique sous forme du produit de réaction en milieu aqueux, à température ambiante, de l'acide undécylénique avec au moins une macromolécule organique hydrophile possédant des groupements alcool primaire et/ou amine primaire, ayant une masse moléculaire supérieure à 2000 Daltons, comme agent actif à la fabrication d'une composition cosmétique ou pharmaceutique, à activité anti-microbienne, anti-bactérienne, ou anti-fongique, en particulier à une proportion comprise entre 0,1 et 5 % en poids par rapport au poids total de la composition.

Comme il a été dit précédemment, le dérivé acide undécylénique - macromolécule organique hydrophile selon la présente invention présente les nouvelles propriétés inattendues anti-pelliculaire, anti-acnéique, déodorante et anti-sudorale ainsi que comme agent conservateur principal ou auxiliaire dans le cas de formulations cosmétiques ou pharmaceutiques, en particulier dermatologiques. Elles peuvent être également utilisées comme agent hydratant ou émulsionnant.

Le procédé de fabncation des dérivés de l'acide undécylénique comprend la mise en réaction en milieu aqueux, à la température ambiante, de l'acide undécylénique sous forme réactive avec au moins une macromolécule organique hydrophile possédant des groupements alcool primaire et/ou amine primaire.

Dans la descnption et les revendications, les termes « sous forme réactive » impliquent que l'acide undécylénique est sous une forme capable de réagir avec ladite macromolécule à la température ambiante. Une telle forme réactive bien connue à l'homme de l'art consiste en particulier en un dérivé halogéné ou en un anhydride d'acide.

Selon un mode de réalisation avantageux du procédé, on réalise tout d'abord la dissolution ou la mise en suspension dans ledit milieu aqueux de la macromolécule organique hydrophile précitée sous agitation vigoureuse jusqu'à obtention d'une solution ou d'une suspension homogène, puis l'acide undécylénique sous ladite forme réactive est ajouté sous agitation, à la température ambiante.

La réaction est prolongée pendant une période de temps suffisante bien connue à l'homme de l'art, qui en général variera entre environ 30 min et environ 5 h, typiquement environ 2 h.

Selon une variante de réalisation particulière, à la fin de la réaction, on peut avantageusement neutraliser le milieu réactionnel avec une base forte ou faible jusqu'à un pH voisin de la neutralité.

Selon une autre variante de réalisation avantageuse, le produit de réaction formant le dérivé de l'acide undécylénique et de la macromolécule, comprenant des liaisons covalentes, ainsi que des liaisons de type ionique et hydrophobe et une haute teneur en acides gras peut être séché, préférentiellement par lyophilisation.

Selon une autre variante de réalisation avantageuse, la réaction a lieu avec un rapport relatif en pourcentage en poids macromolécule/acide gras compris entre 25/75 et 98/2, pour obtenir une proportion relative d'acide undécylénique par rapport à la macromolécule organique hydrophile comprise entre environ 10 et environ 75 % en poids.

Il est à noter que le séchage par la lyophilisation permet d'obtenir des dérivés comprenant la macromolécule organique hydrophile précitée sur laquelle ont été fixés jusqu'à 75 % d'acide gras et qui ne présentent pas un aspect huileux, ce qui est tout à fait remarquable.

Selon une autre variante de réalisation avantageuse du procédé de l'invention, le pH de réaction peut être compris entre 5 et 12. Selon une variante de réalisation particulière, il est préféré d'amener le pH de la solution ou suspension aqueuse contenant la macromolécule à une valeur supérieure à environ 8 avant d'ajouter l'acide undécylénique sous sa forme réactive.

D'autres variantes de réalisation du procédé, notamment concernant la nature de la macromolécule résultent de la description précédente et de celle qui va suivre ainsi que des revendications.

D'autres buts, caractéristiques et avantages de l'invention apparaîtront clairement à la lumière de la description explicative qui va suivre faite en référence à plusieurs exemples de l'invention donnés simplement à titre d'illustration et qui ne sauraient donc en aucune façon limiter la portée de l'invention. Tous les pourcentages sont donnés en poids, sauf indication contraire.

Exemple 1 de l'invention

A - Fabrication du dérivé acide undécylénique - protéines de blé

25 g de protéines de blé de haut poids moléculaire (Tritisol, CRODA) sont mis en suspension dans 500 ml d'eau déminéralisée sous agitation constante. Après dissolution complète du polymère, le pH est ajusté à 9,5 et 75 g de chlorure d'acide undécylénique sont ajoutés au mélange.

Le greffage se déroule à la température ambiante lors d'un laps de temps compris entre 1 h et 5 h, au cours duquel le pH chute de 9,5 à une valeur inférieure à 1.

Après réaction complète, le pH est ajusté aux environs de 7,5 avec de l'hydroxyde de sodium concentré (12N). Cette neutralisation se fait lentement, sous agitation constante.

Après stabilisation du pH, la solution est lyophilisée. Le lyophilisat est ensuite broyé, mis en sachets individuels scellés, puis stérilisé par rayonnement bêta (15 k Gray) ou gamma (25 k Gray).

Le rayonnement gamma est particulièrement intéressant dans le cas des dérivés de l'invention ainsi fabriqués, car il a été vérifié qu'il n'altère ni la structure des molécules composant ce macrocomplexe, ni les insaturations portées par les molécules d'acide undécylénique.

B - Dérivé acide undécylénique - protéines de soie

On procède comme en A ci-dessus, mais en utilisant des protéines de soie de poids moléculaire moyen 10 000 D disponibles dans le commerce.

Exemple 2 de l'invention

Préparation du dérivé acide undécylénique - extrait d'algue rose

On procède comme décrit à l'exemple 1, si ce n'est que l'on met à réagir 50 g d'extrait d'algue rose micronisée disponible dans le commerce de poids moléculaire moyen 5 000 D avec 50 g de chlorure d'acide undécylénique.

Exemple 3 de l'invention

Préparation du dérivé acide undécylénique - collagène extrait de peau de poissons

On procède comme décrit à l'exemple 1, si ce n'est que l'on utilise 25 g de collagène extrait de peau de poissons de poids moléculaire moyen 100 000 D que l'on met à réagir avec 75 g de chlorure d'acide undécylénique.

Exemple 4 de l'invention

Préparation du dérivé de l'exemple 1 sous une autre forme basique

On procède comme décrit à l'exemple 1, mais la neutralisation est effectuée soit par la potasse alcaline (KOH) concentrée, soit encore par un tampon tel que carbonate, phosphate, borate ou ammoniaque.

Exemple 5 de l'invention

Préparation du dérivé de l'exemple 1 sous une autre forme basique

On procède comme décrit à l'exemple 1, mais la neutralisation est effectuée soit par l'hydroxyde d'aluminium (AlOH$_3$), soit par l'hydroxyde carbonate de zinc (Zn(OH)$_2$ ZnCO$_3$).
Cette neutralisation peut être totale ou partielle et, dans ce dernier cas, la fin de la neutralisation est réalisée par une base chimique comme NaOH ou KOH.

Exemple 6 de l'invention

Dérivé de l'exemple 1 neutralisé par une amine

On procède comme décrit à l'exemple 1, si ce n'est que l'on réalise la neutralisation avec une amine telle que la monoéthanolamine, la diéthanolamine la triéthanolamine.

Exemple 7 de l'invention

A - Préparation du dérivé acide undécylénique - xanthane

On procède comme décrit à l'exemple 1, si ce n'est que l'on utilise 25 g de xanthane de poids moléculaire moyen 1 000 000 D que l'on met à réagir avec 38 g de chlorure d'acide undécylénique et 38 g de chlorure d'acide stéarique.
Après la fin de la réaction, la neutralisation s'effectue par ajout d'une amine, par exemple la triéthanolamine.

B - Dérivé acide undécylénique - kératine

On procède comme décrit en A ci-dessus, mais en utilisant de la kératine disponible dans le commerce de poids moléculaire moyen 120 000 D.

Exemple 8 de l'invention

Préparation d'un dérivé acide undécylénique - ADN

On procède comme décrit à l'exemple 1, mais on utilise 25 g d'ADN de haut poids moléculaire (poids moléculaire moyen égal à 500 000 D disponible dans le commerce) mis à réagir avec 38 g de chlorure d'acide undécylénique et 38 g de chlorure d'acide laurique.
Après la fin de la réaction, la neutralisation s'effectue par ajout de soude caustique.

Exemple 9 de l'invention

Mise en évidence d'une activité anti-pelliculaire

1 - Tests réalisés

L'espèce fongique mise en cause dans la composante microbienne des états pelliculaires est la levure appelée Pityrosporum ovale. Dans cette optique, un certain nombre de dérivés de l'invention à base d'acide undécylénique ont été étudiés pour leur activité fongistatique vis-à-vis de Pityrosporum ovale.

Matériel et méthodes

La détermination de l'activité fongistatique du produit a été réalisée par la méthode des dilutions en milieu gélosé.

Technique

Des quantités décroissantes du produit à étudier en solution dans l'eau distillée sont incorporées sous un même volume (1 ml) à un même volume de milieu de culture gélosé fondu et ramené à 45°C. Après mélange soigneux en boîtes de Petri et solidification du milieu, le germe test est ensemencé en surface à l'öse calibrée 4 mm$^3$ en une strie. Après incubation de 72 h à 30°C, la concentration minimale inhibitrice (CMI) est donnée par la plus faible concentration de produit qui inhibe la croissance microbienne.

Produits étudiés

- dérivé de l'invention :

  - protéines de soie/undécylénique de l'exemple 1B
  - collagène/undécylénique de l'exemple 3
  - kératine/undécylénique de l'exemple 6B
  - extrait d'algues roses/undécylénique de l'exemple 2

Pour les essais, une solution mère à 50 mg/ml en eau distillée stérile a été préparée extemporanément.

Souche étudiée

La souche étudiée est une souche référencée, distribuée par le "Centraalbureau voor Schimmelcultures de Baarn (CBS) (Hollande) : Pityrosporum ovale CBS 1878".

Milieu de culture

Le milieu ATCC n 42 a été utilisé pour l'entretien de la souche, la production de l'inoculum d'essai et la détermination de l'activité microbiostatique. Sa formule est la suivante :

| | |
|---|---|
| gélose de Sabouraud déshydratée, q.s.p | 1 000 ml |
| polysorbate 40 | 10,0 g |
| glycérol mono-oléate | 2,5 g |
| agar-agar | 5,0 g |
| eau distillée | 1 000 ml |
| stérilisation à l'autoclave 20 min à 120°C | |

L'inoculum d'essai était constitué d'une suspension en soluté isotonique stérile des levures (10$^7$ levures/ml) obtenues sur ce milieu après deux repiquages successifs.

conditions d'incubation

Incubation en aérobiose, 72 h à 30°C.

Résultat

Les concentrations minimales pour lesquelles il y a inhibition du développement de Pityrosporum ovale sont les suivantes :

| | |
|---|---|
| protéines soie/undécylénique de l'exemple 1B | 0,3 % soit 3 mg/ml |
| collagène/undécylénique de l'exemple 3 | 0,1 % soit 1 mg/ml |
| kératine/undécylénique de l'exemple 6B | 0,2 % soit 2 mg/ml |
| extrait d'algues roses/undécylénique de l'exemple 2 | 0,2 % soit 2 mg/ml |

2 - Formulations

L'activité anti-pelliculaire des dérivés de l'invention de l'acide undécylénique est particulièrement intéressante dans le domaine des shampooings. Pour cela, différentes formulations de shampooing ont été réalisées et le dérivé de l'invention a été incorporé à une concentration minimale de 0,3 %.

Exemple 10

Shampooing familial

| A | TEXAPON N 40® | 40 | HENKEL |
|---|---|---|---|
| | COMPERLAN KD® | 2 | HENKEL |
| | | | |
| B | Dérivé undécylénique - protéines de soie de l'exemple 1B | 0,3 | COLETICA |
| | Chlorure de sodium | 1,5 | |
| | Eau qsp | 100 | |
| | | | |
| C | Phénonip® | 0,5 | SEPPIC |
| | Bactéricide MB® | 0,5 | DRAGOCO |

A à 20°C
B à 80°C
Mettre B dans A sous agitation
Refroidir jusqu'à 20°C
Ajouter C.

Exemple 11

Shampooing doux

| A | TWEEN20® | 10 | ICI |
|---|---|---|---|
| | TEGO BETAINE L7® | 10 | GOLDSCHMIDT |
| | G 1821® | 3 | ICI |
| | | | |
| B | Dérivé undécylénique - collagène de l'exemple 3 | 0,5 | COLETICA |
| | Eau qsp | 100 | |
| | | | |
| C | Phénonip® | 0,5 | SEPPIC |
| | Bactéricide MB® | 0,5 | DRAGOCO |

(suite)

|  | |  | |
|---|---|---|---|
| Homogénéiser A sous agitation à 20°C | | | |
| Homogénéiser B à 80°C | | | |
| Mettre B dans A sous agitation | | | |
| Refroidir jusqu'à 20°C | | | |
| Ajouter C. | | | |

Exemple 12

Shampooing nacré

| A | TEXAPON N 40® | 40 | HENKEL |
|---|---|---|---|
|  | COMPERLAN KD® | 2 | HENKEL |
|  | EUPERLAN PK 771® | 4 | HENKEL |
| B | Dérivé undécylénique - kératine | 0,5 | COLETICA |
|  | Chlorure de sodium | 1,5 | |
|  | Eau qsp | 100 | |
| C | Phénonip® | 0,5 | SEPPIC |
|  | Bactéricide MB® | 0,5 | DRAGOCO |
| Homogénéiser A sous agitation à 20°C | | | |
| Homogénéiser B à 80°C | | | |
| Mettre B dans A et refroidir sous agitation | | | |
| Ajouter C à 20°C. | | | |

Lors de la réalisation de ces différentes formules, les inventeurs ont remarqué que les macromolécules ainsi modifiées ne précipitaient pas lorsqu'elles étaient incorporées dans ces différentes formules de shampooing. Ceci n'est pas le cas de macromolécules de haut poids moléculaire, qui ont tendance à perdre leurs structures tertiaire et quaternaire en présence de détergents et, ainsi, à précipiter dans un milieu "shampooing" ou "gel douche". Les dérivés de l'invention incorporés dans de telles formules apportent donc à la fois une action anti-pelliculaire, ainsi qu'une nouvelle forme d'utilisation des macromolécules dans des milieux concentrés en détergents.

Exemple 13

Mise en évidence de l'activité anti-acnéique

1 - Tests réalisés

Les espèces bactériennes les plus fréquemment mises en cause dans la composante bactérienne de l'acné vulgaire sont les micro-organismes suivants : Staphylococcus aureus, Staphylococcus epidermidis et Propionibacterium acnes. Dans cette optique, un certain nombre de dérivés de l'invention à base d'acide undécylénique ont été étudiés pour leur activité bactériostatique vis-à-vis des trois micro-organismes cités précédemment.

Matériel et méthodes

La détermination de l'activité microbiostatique du produit a été réalisée par la méthode des dilutions en milieu gélosé.

Technique

Des quantités décroissantes du produit à étudier en solution dans l'eau distillée sont incorporées sous un même volume (1 ml) à un même volume (9 ml) de milieu de culture gélosé fondu et ramené à 45°C. Après mélange soigneux en boîtes de Petri et solidification du milieu, le germe test est ensemencé en surface à l'öse calibrée (4 mm$^3$) en une

strie.

Après incubation dans des conditions (température, atmosphère et durée) fonction de la souche microbienne étudiée, la concentration minimale inhibitrice (CMI) est donnée par la plus faible concentration de produit qui inhibe la croissance microbienne.

Produits étudiés

dérivés de l'invention :

- collagène/acide undécylénique de l'exemple 1B
- extrait d'algues roses/acide undécylénique de l'exemple 2

Par ailleurs, l'acide caprylique (huit carbones) et ses dérivés ont déjà été décrits dans la littérature (Int. J. of Cosmetic Science II, 253-258 (1989) pour leur action inhibitrice sur les souches bactériennes responsables de l'acné. Nous avons donc réalisé un greffage extrait d'algues roses/acide caprylique de l'exemple 2 afin de comparer l'action des dérivés de l'invention à base d'acide undécylénique de celle des dérivés de l'invention à base d'acide caprylique.

Pour les essais, une solution mère à 50 mg/ml en eau distillée stérile a été préparée extemporanément.

Souches étudiées

Les 3 souches étudiées sont des souches référencées, distribuées par le Service de la Collection de l'Institut Pasteur de Paris (CIP) :

1 - Staphylococcus aureus CIP 53154
2 - Staphylococcus epidermidis CIP 53124
3 - Propionibacterium acnes CIP 53117

Milieux de culture

a) Milieux d'entretien des souches :

- gélose aux peptones de caséine et soja Difco : pour S. aureus et S. epidermidis
- bouillon de Schaedler (IPP) pour P. acnes

b) Milieux pour la production de l'inoculum d'essai :

- bouillon aux peptones de caséines et soja Merck pour S. aureus et S. epidermidis
- bouillon de Schaedler (IPP) pour P. acnes

c) Milieux de détermination de l'activité microbiostatique :

- pour S. aureus et S. epidermidis : gélose de Mueller-Hinton (IPP)
- pour P. acnes : gélose de Wilkins-Charlgreen (Oxoid)

Conditions d'incubation :

- pour S. aureus et S. epidermidis : incubation en aérobiose, 24 h à 37°C
- pour P. acnes : incubation en jarre anaérobie, 72 h à 37°C.

Résultats

Les concentrations minimales pour lesquelles il y a inhibition du développement de chacune des bactéries précitées sont les suivantes :

| | Staphylococcus aureus | Staphylococcus epidermidis | Propionibacterium acnes |
|---|---|---|---|
| Collagène/undécylénique (ex. 3) | 0,2 % | ND | 0,5 % |

(suite)

|  | Staphylococcus aureus | Staphylococcus epidermidis | Propionibacterium acnes |
|---|---|---|---|
| Extrait d'algues roses/ undécylénique (ex. 2) | 0,5 % | 0,7 % | 0,2 % |
| Extrait d'algues roses/ caprylique | 4 % | 4 % | 1% |

Comme nous pouvons le constater, les dérivés de l'acide undécylénique sont beaucoup plus efficaces que les dérivés capryliques.

2 - Formulations

L'activité anti-acnéique des dérivés de l'invention de l'acide undécylénique est particulièrement intéressante dans le domaine des laits ou des crèmes pour les soins du visage. Pour cela, différentes formulations de laits et de crèmes ont été réalisées et le dérivé de l'invention a été utilisé en tant qu'émulsionnant unique à des concentrations minimales de 1 %.

Exemple 14

Crème anti-acnéique

| A | Dérivé de l'invention | 1,00 % | COLETICA |
|---|---|---|---|
|  | CARBOPOL 940® | 0,50 % | POLYPLASTIC |
|  | Eau distillée | 82,5 % |  |
|  | Propylèneglycol | 2,0 % | HULS |
| B | Cire d'abeille blanche | 3,00 % | LA CERESINE |
|  | Lanette C16 C18® | 3,00 % | HENKEL |
|  | DRAGOXAT® | 3,00 % | DRAGOCO |
|  | PCL solide® | 3,00 % | DRAGOCO |
| C | GERMALL II® | 0,30 % | SEPPIC |
|  | Phénonip® | 0,50 % | SIPCA |
|  | TEA (triéthanolamine) | 1,00 % |  |
|  | Silicone Abil 100 | 0,50 % | GOLDSCHMIDT |

Les phases A et B sont chauffées séparément jusqu'à 80°C, puis B est ajouté à A sous forte agitation. L'ensemble est laissé à refroidir sous agitation modérée. A 20°C, la phase C est rajoutée sous agitation.

Exemple 15

Crème anti-acnéique

| A | Dérivé de l'invention | 1,00 % | COLETICA |
|---|---|---|---|
|  | Propylèneglycol | 2,00% | HULS |
|  | Eau distillée | 54,50% |  |
| B | Cire d'abeille blanche | 3,00 % | LA CERESINE |
|  | Lanette 14® | 3,00 % | HENKEL |
|  | DRAGOXAT® | 3,00 % | DRAGOCO |
|  | PCL solide® | 3,00 % | DRAGOCO |

(suite)

| C | Farine de caroube | 0,5 % | SANOFI |
|---|---|---|---|
|   | Eau distillée | 29 % |   |
|   |   |   |   |
| D | Phénonip® | 0,50 % | SIPCA |
|   | Bactéricide MB® | 0,50 % | DRAGOCO |

C est dissous à froid. A et B sont chauffés séparément à 80°C. Puis C est ajouté à A. Lorsque A + C est revenu à 80°C, B est ajouté sous forte agitation. L'ensemble est laissé à refroidir sous agitation lente. A 20°C, la phase D est rajoutée sous agitation.

## Exemple 16

### Lait anti-acnéique

| A | Huile de bourrache | 6 | CRODA |
|---|---|---|---|
|   | Huile de jojoba | 2 | HENKEL |
|   | Arlamol HD | 5 | ICI |
|   | Alcool cétylique | 1 | HENKEL |
|   |   |   |   |
| B | Dérivé de l'invention | 1 | COLETICA |
|   | Eau distillée | 55 |   |
|   |   |   |   |
| C | Farine de caroube | 0,5 | SANOFI |
|   | Eau distillée | 29 |   |
|   |   |   |   |
| D | Phénonip® | 0,5 | SIPCA |
|   | Bactéricide MB® |   | DRAGOCO |
|   |   | 100% |   |

C est dissous à froid. A et B sont chauffés séparément à 80°C. Puis C est ajouté à A. Lorsque A + C est revenu à 80°C, B est ajouté sous forte agitation. L'ensemble est laissé à refroidir sous agitation lente. A 20°C, la phase D est rajoutée sous agitation.

## Exemple 17

### Lait démaquillant

| A | Dérivé de l'invention | 1 % | COLETICA |
|---|---|---|---|
|   | Eau | 55 % |   |
|   |   |   |   |
| B | Huile de vaseline | 6 % | LASERSON ET SABETAY |
|   | Cétiol J 600® | 2 % | HENKEL |
|   | Cire d'abeille blanche | 2 % | LA CERESINE |
|   | Alcool cétylique | 1 % | HENKEL |
|   | Acide stéarique | 1 % | AKZO |
|   |   |   |   |
| C | Caroube | 0,3 % | SANOFI |
|   | Eau distillée | 34 % |   |

(suite)

| D | Phenonip® | 0,5 % | SIPCA |
|---|---|---|---|
| | Bactéricide MB® | 0,5 % | DRAGOCO |
| | Propylèneglycol | 0,5 % | HULS |

C est dissous à froid. A et B sont chauffés séparément à 80°C. Puis C est ajouté à A. Lorsque A + C est revenu à 80°C, B est ajouté sous forte agitation. L'ensemble est laissé à refroidir sous agitation lente. A 20°C, la phase D est rajoutée sous agitation.

## Exemple 18

### Mise en oeuvre d'une activité déodorante et anti-sudorale

Il existe trois moyens pour lutter efficacement contre les odeurs corporelles émises au niveau des aisselles, où les glandes eccrines et apocrines sont présentes :

- les bactéricides qui représentent 80 % du marché. Ils agissent après l'émission de la sueur (inodore à l'émission), en détruisant les bactéries qui la dégradent et entraînent de mauvaises odeurs.
- les anti-transpirants qui agissent directement sur l'émission de la sueur dont ils diminuent la sécrétion.
- les capteurs d'odeurs qui neutralisent et absorbent les odeurs dès qu'elles se forment.

Comme cela a été démontré dans le brevet FR-7101431, les dérivés des acides undécylénique ou caprylique ont des propriétés déodorantes et anti-sudorales permettant d'empêcher le développement de souches bactériennes et le développement d'odeurs corporelles désagréables.

De même, les dérivés de l'acide undécylénique neutralisé par des sels d'aluminium ou de zinc, tel que décrit dans l'exemple 5, permettent d'accroître le potentiel anti-sudoral de ces dérivés.

Les dérivés de l'invention, qui sont des macromolécules polysaccharidiqucs ou protéiques à structure greffée à l'acide undécylénique, permettent d'obtenir des propriétés similaires.

Cependant, plus que des molécules bactéricides, les dérivés de l'invention sont de véritables réservoirs à acide undécylénique, qui n'interviennent que lorsqu'ils sont sollicités par les bactéries. En se développant, celles-ci dégradent les dérivés de l'invention et permettent la programmation de leur propre destruction.

Formulation d'une lotion déodorante et anti-sudorale :

| Dérivé de l'invention | 2 % |
|---|---|
| Eau | 18 % |
| Alcool à 95° | 80 % |

## Exemple 19

### Mise en oeuvre d'une activité des dérivés de l'invention en tant qu'agent conservateur principal ou auxiliaire dans une formulation cosmétique ou dermatologique

Dans une des formules de lait et de crèmes précédentes, les conservateurs ont été retirés. La présence de pollution bactérienne a été recherchée jusqu'à 50 jours après fabrication des laits et des crèmes, pour des formulations contenant de 1 % à 1,5 % de dérivé de l'invention (couplages algues roses/acide undécylénique, gomme d'acacia/acide undécylénique, collagène marin/acide undécylénique).

Aucune pousse bactérienne n'a été relevée pendant cette période.

En raison de cette activité anti-bactérienne très intense, une série de tests de résistance à la contamination microbienne, également appelés "challenge tests" a été réalisée sur des solutions aqueuses de dérivés de l'invention à 1 %, 2 % et 3 %.

### Matériel et méthodes

La résistance à la contamination microbienne des trois solutions étudiées a été déterminée selon les prescriptions de la Pharmacopée Britannique 1988, vol.II, ppA200-A203 ; Efficacy of antimicrobial preservatives in pharmaceutical products.

1. Produit étudié - Préparation des solutions

- Dérivés réf. Arun, algue rose couplée chaînes undécyléniques. Pour l'essai, trois solutions en eau distillée stérile ont été préparées extemporanément titrant respectivement 1 % m/V, 2 % m/V et 3 % m/V. La dispersion du dérivé de l'invention ARUN a été obtenue par agitation magnétique dans un bain-marie régulé à 50 ± 1°C.

2. Souches microbiennes

Les souches microbiennes incluses dans l'essai sont les souches préconisées par la Pharmacopée Britannique 1988 pour l'essai d'efficacité des agents de conservation antimicrobiens dans les préparations pour usage topique.

Staphylococcus aureus ATCC 6538 (CIP 53156)
Pseudomonas aeruginosa ATCC 9027 (CIP 82118)
Candida albicans ATCC 10231 (CIP 4872)
Aspergillus niger ATCC 16404 (CIP 1431-83)

Ces souches ont été utilisées, pour la préparation des inoculum, dans les conditions prescrites. Pour chaque souche, l'inoculum a été préparé extemporanément avant l'inoculation des échantillons. La population en germes viables dans les échantillons contaminés au temps 0 a été déterminée par dénombrement de chacun de ces inoculum.

3. Procédé

3.1 Inoculation des échantillons

Les solutions de produit ont été réparties à raison de 100 ml en flacons de verre borosilicaté bouchés à vis de 150 ml.
Le premier jour (JO), chacun de ces échantillons a été ensemencé avec 1 ml de suspension inoculum (renfermant $5.10^7$ à $5.10^8$ germes viables par ml) de l'un des 4 micro-organismes étudiés.

3.2 Suivi des échantillons

Les échantillons contaminés ont été conservés à 25 ± 1°C. Après 48 h, 7, 14, 21 et 28 jours de conservation, les germes viables ont été dénombrés. A partir du 7e jour, la recherche des germes contaminants a été effectuée dans 1 ml de produit contaminé lorsque la population obtenue lors du dénombrement précédent avait été trouvée inférieure à 10 UFC/ml.

3.3 Dénombrement des germes viables

Le dénombrement des germes viables, dans les inoculum et les échantillons contaminés, a été effectué par la méthode des dilutions en milieu gélosé. Pour les dénombrements dans les échantillons contaminés, selon les micro-organismes étudiés, deux diluants ont été utilisés en fonction des résultats des essais de validation effectués au préalable :

- pour P. aeruginosa et S. aureus: eau peptonée salée dont la formule est la suivante :

| Peptone | 1,0 g |
|---|---|
| NaCl | 8,5 g |
| Eau distillée | 1 000 ml |

- pour C. albicans et A. niger neutralisant à usage général décrit par la Pharmacopée Française Xe ed. VIII.10A. Sa formule est la suivante :

| Polysorbate 80 | 30 g |
|---|---|

(suite)

| | |
|---|---|
| Lécithine de jaune d'oeuf | 3 g |
| Histidine HCl | 1 g |
| Peptone de caséine | 1 g |
| Chlorure de sodium | 4,3 g |
| Phosphate monopotassique | 3,56 g |
| Phosphate disodique dihydraté | 7,23 g |
| Eau distillée | 1 000 ml |

Pour la recherche des germes contaminants, 1 ml de produit a été introduit dans 100 ml de bouillon aux peptones de caséine et de soja additionnés de 10 ml de diluant neutralisant. Après 48 h d'incubation à 37°C, un isolement sur milieu gélosé aux peptones de caséine et de soja a été réalisé. L'absence de colonies du contaminant considéré sur le milieu d'isolement permettait de conclure à l'absence de ce germe dans 1 ml de produit.

Résultats

Les résultats obtenus sont présentés dans les tableaux 1, 2 et 3 ci-après.

Vis-à-vis de S. aureus, les trois solutions aqueuses de dérivé ARUN possèdent une très bonne résistance à la contamination, puisque, 48 h après la contamination, une diminution supérieure à 5 log de la population initiale est observée. Au 7$^e$ jour, ces bactéries ne sont plus retrouvées dans 1 ml de produit. Cette efficacité est conforme aux critères requis par la Pharmacopée Britannique (BP).

Vis-à-vis de C. albicans, la résistance à la contamination est aussi excellente, puisqu'au 7$^e$ jour ce germe n'est plus retrouvé dans 1 ml de produit. Cette efficacité est supérieure aux critères requis par la BP (diminution de 2 log en 14 jours).

Vis-à-vis des spores d'A. niger, la résistance à la contamination des trois solutions est proportionnelle à la concentration en ARUN. Pour la solution à 1 %, l'efficacité n'atteint pas le niveau requis par la BP (diminution de 1 log en place des 2 log requis) mais est conforme aux exigences de la Pharmacopée Française. Les solutions à 2 et 3 % possèdent par contre une efficacité conforme aux critères de la BP. Pour les trois concentrations, la décroissance se poursuit lentement jusqu'au dernier jour de l'essai.

Vis-à-vis de P. Aeruginosa, les trois solutions sont inefficaces. Dès la 48$^e$ h, une augmentation d'environ 1 log de la population initiale est en effet observée. La population se stabilise à partir du 14$^e$ jour d'observation.

Aussitôt après préparation, le pH des solutions est de :

- pour 1 % : pH 6,53
- pour 2 % : pH 6,73
- pour 3 % : pH 6,90

Afin d'écarter l'hypothèse de l'influence négative d'un pH voisin de la neutralité sur l'efficacité du dérivé ARUN vis-à-vis de P. aeruginosa, l'essai a été répété sur une solution à 2 % m/V ajustée à pH 5,42 par addition d'acide chlorhydrique. Les résultats obtenus avec cette solution sont analogues à ceux rapportés précédemment :

- J0 : 2.7.10$^6$ UFC/ml
- J2 : 1.4.10$^7$ UFC/ml
- J7 : 4.5.10$^7$ UFC/ml

L'inefficacité du produit n'est donc pas liée à un pH trop proche de la neutralité.

Tableau 1

| Evolution de la population microbienne ajoutée dans une solution à 1 % m/V de dérivé de l'invention ARUN (population en UFC/ml). | | | | | | |
|---|---|---|---|---|---|---|
| Solution aqueuse à 1 % m/V d'ARUN | | | | | | |
| | 0j | 48h | 7j | 14j | 21j | 28j |
| S. aureus | 1,7.10$^8$ | <5 | 0 | 0 | 0 | 0 |

Tableau 1   (suite)

| Solution aqueuse à 1 % m/V d'ARUN | | | | | |
|---|---|---|---|---|---|
| | 0j | 48h | 7j | 14j | 21j | 28j |
| P. aeruginosa | $3,1.10^8$ | $2,1.10^7$ | $1,3.10^7$ | $>5.10^7$ | $6,0.10^7$ | $6,0.10^7$ |
| C. albicans | $8,4.10^5$ | $<5$ | 0 | 0 | 0 | 0 |
| A. niger | $9,0.10^5$ | $5,0.10^4$ | $9,6.10^4$ | $6,0.10^4$ | $5,3.10^4$ | $2,9.10^4$ |

Tableau 2

| Evolution de la population microbienne ajoutée à une solution à 2 % m/V de dérivé de l'invention ARUN (population en UFC/ml). | | | | | | |
|---|---|---|---|---|---|---|
| Solution aqueuse à 2 % m/V d'ARUN | | | | | | |
| | 0j | 48h | 7j | 14j | 21j | 28j |
| S. aureus | $1,7.10^8$ | $<5$ | 0 | 0 | 0 | 0 |
| P. aeruginosa | $3,1.10^8$ | $1,8.10^7$ | $1,9.10^7$ | $>4.10^7$ | $7,7.10^7$ | $7,1.10^7$ |
| C. albicans | $8,4.10^5$ | $<5$ | 0 | 0 | 0 | 0 |
| A. niger | $9,0.10^5$ | $3,6.10^3$ | $6,1.10^3$ | $2,7.10^3$ | $2,8.10^3$ | $9,5.10^2$ |

Tableau 3

| Evolution de la population microbienne ajoutée dans une solution à 3 % m/V de dérivé de l'invention ARUN (population en UFC/ml). | | | | | | |
|---|---|---|---|---|---|---|
| Solution aqueuse à 3 % m/V d'ARUN | | | | | | |
| | 0j | 48h | 7j | 14j | 21j | 28j |
| S. aureus | $1,7.10^8$ | $<5$ | 0 | 0 | 0 | 0 |
| P. aeruginosa | $3,1.10^8$ | $5,4.10^6$ | $1,7.10^7$ | $1,9.10^7$ | $1,9.10^7$ | $1,7.10^7$ |
| C. albicans | $8,4.10^5$ | $<5$ | 0 | 0 | 0 | 0 |
| A. niger | $9,0.10^5$ | $6,4.10^2$ | $7,1.10^2$ | $4,2.10^2$ | $2,5.10^2$ | $1,2.10^2$ |

Conclusion

Les solutions aqueuses de dérivés ARUN à 1, 2 et 3 % possèdent une bonne résistance à la contamination par Staphylococcus aureus (bactérie à Gram positif), Candida albicans (levure) et de Aspergillus niger (moisissure).

Pour S. aureus et C. albicans une contamination initiale de $10^6$ germes viables/ml est réduite de plus de 5 log en 48 h. Pour A. niger (inoculé à l'état de spores) l'effet observé est proportionnel à la concentration de dérivé de l'invention ARUN: 48 h après l'inoculation, la population initiale est réduite de 1 log avec 1 % m/V, de 2 log avec 2 % m/V et de 3 log avec 3 % m/V.

Par contre, le dérivé de l'invention ARUN n'a aucune activité vis-à-vis de Pseudomonas aeruginosa (bactérie à Gram négatif).

Le dérivé de l'invention ARUN, introduit dans une formulation pour ses propriétés cosmétologiques, pourrait jouer un rôle dans la conservation microbienne de cette formulation à condition de lui adjoindre un conservateur efficace vis-à-vis des bactéries à Gram négatif.

A noter cependant que les mêmes tests réalisés sur des formulations de crèmes ou de laits donnent des résultats encore plus positifs, c'est-à-dire que Pseudomonas aeruginosa disparaît totalement d'un milieu enrichi. La formulation des produits cosmétiques semble donc être un point très important dans la mise en oeuvre des capacités anti-bactériennes des dérivés.

En effet, elle peut permettre d'améliorer les propriétés intrinsèques du dérivé de l'invention, tout en lui permettant d'agir en tant que conservateur exclusif dans une composition cosmétique.

Par conséquent, les dérivés de l'invention réalisés en associant des macromolécules hydrophiles à des chaînes d'acide undécylénique permettent d'obtenir à la fois des actifs autoprotégés et des systèmes conservateurs pouvant être utilisés seuls ou en complément d'autres conservateurs plus classiques tels que les parabènes.

Conclusions

La réalisation de couplages macromolécules hydrophiles/acide undécylénique permet d'aboutir à des composés naturels, biodégradables biocompatibles, de toxicités orale, cutanée et oculaire nulles.

Ces composés ont des propriétés émulsionnantes et permettent également de formuler des macromolécules dans des milieux très dénaturants comme le sont les shampooings ou les gels douches.

Les substantivités obtenues en sont accrues et l'hydratation qui résulte de leur utilisation dans des crèmes ou des laits s'en trouve accélérée.

De plus, les activités anti-bactérienne et anti-fongique de l'acide undécylénique sont toujours fortement présentes dans les dérivés de l'invention, ce qui permet de les utiliser en tant qu'actifs anti-pelliculaire, anti-acnéique, anti-sudoral ou déodorant.

Leurs activités permettent aussi de penser à leur utilisation dans des formulations en tant que conservateur principal ou auxiliaire.

## Revendications

1. Utilisation d'un dérivé de l'acide undécylénique sous forme du produit de réaction en milieu aqueux, à température ambiante, de l'acide undécylénique avec au moins une macromolécule organique hydrophile possédant des groupements alcool primaire et/ou amine primaire, ayant une masse moléculaire supérieure à 2 000 Daltons, comme agent anti-microbien, ou anti-bactérien ou anti-fongique.

2. Utilisation d'un dérivé de l'acide undécylénique sous forme du produit de réaction en milieu aqueux, à température ambiante, de l'acide undécylénique avec au moins une macromolécule organique hydrophile possédant des groupements alcool primaire et/ou amine primaire, ayant une masse moléculaire supérieure à 2 000 Daltons, comme agent actif pour la fabrication d'une composition cosmétique ou pharmaceutique, à activité anti-microbienne, anti-bactérienne ou anti-fongique, en particulier à une proportion en poids dudit agent actif entre 0,1 et 5 % en poids par rapport au poids total de la composition.

3. Utilisation selon la revendication 1 ou 2, caractérisée en ce qu'il s'agit d'un agent conservateur principal ou auxiliaire d'une composition cosmétique ou pharmaceutique, en particulier à une proportion comprise entre 0,1 et 5% en poids, de préférence entre environ 1 et environ 3 % en poids.

4. Utilisation selon la revendication 1 ou 2, caractérisée en ce qu'il s'agit d'un agent anti-pelliculaire, en particulier à une proportion comprise entre 0,1 et 5 % en poids.

5. Utilisation selon la revendication 1 ou 2, caractérisée en ce qu'il s'agit d'un agent anti-acnéique, en particulier à une proportion comprise entre 0,1 et 5 % en poids.

6. Utilisation selon la revendication 1 ou 2, caractérisée en ce qu'il s'agit d'un agent déodorant et anti-sudoral, en particulier à une proportion comprise entre 0,1 et 5 % en poids.

7. Utilisation selon l'une des revendications précédentes, caractérisée en ce que la proportion relative d'acide undécylénique par rapport à la macromolécule organique hydrophile précitée est de 10 à 75 % en poids, de préférence entre 50 et 75 % en poids.

8. Utilisation selon l'une des revendications précédentes, caractérisée en ce que la macromolécule organique hydrophile précitée présente une masse moléculaire supérieure à 5 000 Daltons, de préférence comprise entre 5 000 Daltons et 1 000 000 Daltons et encore de préférence comprise entre 50 000 Daltons et 150 000 Daltons.

9. Utilisation selon l'une des revendications précédentes, caractérisée en ce que le produit de réaction a subi une étape de lyophilisation.

10. Utilisation selon l'une des revendications précédentes, caractérisée en ce que l'acide undécylénique est mis à réagir sous forme réactive, par exemple sous forme d'halogénure ou sous forme d'anhydride.

11. Utilisation selon l'une des revendications précédentes, caractérisée en ce que la macromolécule organique hydrophile précitée est d'origine animale, végétale ou marine ou de fermentation et est de préférence choisie parmi la kératine, la soie, le collagène, la réticuline, l'élastine, la lactalbumine, la lactoglobuline, la caséine, l'ovalbumine ; l'acide hyaluronique, le chondroïtine-4-sulfate, le chondroïtine-6-sulfate, le dermatane sulfate, l'héparane sulfate, le kératane sulfate ainsi que l'héparine et ses dérivés ou des fractions de masse moléculaire supérieure à 5 000 Daltons ; et protéines de blé, de germes de blé, de maïs, d'avoine, de soja, d'amande, les protéines de poissons, de mollusques, de crustacés, d'algues, l'amylose, l'amylopectine, les glucomannanes, les galactomannanes, les fructosanes, les gommes, les celluloses et dérivés, les alginates, l'agar le carraghénane et les acides nucléiques ADN, ARN ; le xanthane, le gellane, le curdlane, le dextrane.

12. Utilisation selon l'une des revendications précédentes, caractérisée en ce que la macromolécule organique hydrophile précitée comprend des groupements amine primaire tels que les protéines ou les acides ribonucléiques (ADN, ARN).

13. Utilisation selon l'une des revendications précédentes, caractérisée en ce que le dérivé d'acide undécylénique est choisi parmi le groupe consistant du produit de réaction de l'acide undécylénique avec une protéine de blé de haut poids moléculaire, d'extrait de spiruline, du collagène extrait de peau de poissons, de xanthane, d'ADN de haut poids moléculaire, protéines de la soie de haut poids moléculaire, de kératine, de protéines d'au moins 5 000 Daltons extraites d'algues roses, d'un mélange d'ADN de haut poids moléculaire avec un acide gras, en particulier l'acide laurique.

14. Utilisation selon l'une des revendications précédentes, caractérisée en ce que le produit de réaction a subi une neutralisation par une base organique ou minérale, telle qu'une amine ou un hydroxyde de zinc ou d'aluminium.

15. Utilisation selon l'une des revendications précédentes, caractérisée en ce que le dérivé d'acide undécylénique est sous forme du milieu réactionnel neutralisé par une base organique ou minérale, et ayant subi ensuite une étape de lyophilisation.

16. Utilisation selon l'une des revendications précédentes, caractérisée en ce que le dérivé d'acide undécylénique est obtenu par mise en réaction de l'acide undécylénique en mélange avec un autre acide gras saturé ou insaturé, en particulier ayant de 8 à 28 atomes de carbone.

**Patentansprüche**

1. Verwendung eines Derivats von Undecylensäure in Form des Reaktionsprodukts, in wässerigem Medium bei Umgebungstemperatur, von Undecylensäure mit zumindest einem hydrophilen organischen Makromolekül, das primäre Alkohol- und/oder primäre Amin-Gruppen aufweist, und eine Molmasse von mehr als 2000 Dalton hat, als antimikrobielles oder antibakterielles oder antimykotisches Mittel.

2. Verwendung eines Derivats von Undecylensäure in Form des Reaktionsprodukts, in wässerigem Medium bei Umgebungstemperatur, von Undecylensäure mit zumindest einem hydrophilen organischen Makromolekül, das primäre Alkohol- und/oder primäre Amin-Gruppen aufweist, und eine Molmasse von mehr als 2000 Dalton hat, als aktives Mittel zur Herstellung einer kosmetischen oder pharmazeutischen Zusammensetzung mit antimikrobieller, antibakterieller oder antimykotischer Wirksamkeit, insbesondere bei einem Masseverhältnis des aktiven Mittels zwischen 0,1 und 5 Masse-%, bezogen auf die Gesamtmasse der Zusammensetzung.

3. Verwendung nach Anspruch 1 oder 2, dadurch gekennzeichnet, daß es sich um ein Haupt- oder Hilfskonservierungsmittel einer kosmetischen oder pharmazeutischen Zusammensetzung, insbesondere bei einem Verhältnis zwischen 0,1 und 5 Masse-%, vorzugsweise zwischen etwa 1 und etwa 3 Masse-%, handelt.

4. Verwendung nach Anspruch 1 oder 2, dadurch gekennzeichnet, daß es sich um ein Anti-Schuppenmittel, insbesondere bei einem Verhältnis zwischen 0,1 und 5 Masse-%, handelt.

5. Verwendung nach Anspruch 1 oder 2, dadurch gekennzeichnet, daß es sich um ein Anti-Aknemittel, insbesondere bei einem Verhältnis zwischen 0,1 und 5 Masse-%, handelt.

6. Verwendung nach Anspruch 1 oder 2, dadurch gekennzeichnet, daß es sich um ein desodorierendes und schweißhemmendes Mittel, insbesondere bei einem Verhältnis zwischen 0,1 und 5 Masse-%, handelt.

7. Verwendung nach einem der vorhergehenden Ansprüche, dadurch gekennzeichnet, daß das relative Verhältnis von Undecylensäure, bezogen auf das genannte hydrophile organische Makromolekül, 10 bis 75 Masse-%, vorzugsweise zwischen 50 und 75 Masse-%, beträgt.

8. Verwendung nach einem der vorhergehenden Ansprüche, dadurch gekennzeichnet, daß das genannte hydrophile organische Makromolekül eine Molmasse von mehr als 5000 Dalton, vorzugsweise zwischen 5000 Dalton und 1 000 000 Dalton und bevorzugter zwischen 50 000 Dalton und 150 000 Dalton, aufweist.

9. Verwendung nach einem der vorhergehenden Ansprüche, dadurch gekennzeichnet, daß das Reaktionsprodukt einem Lyophilisationsschritt unterworfen wurde.

10. Verwendung nach einem der vorhergehenden Ansprüche, dadurch gekennzeichnet, daß die Undecylensäure in reaktiver Form, beispielsweise in Form eines Halogenids oder in Form eines Anhydrids, umgesetzt wird.

11. Verwendung nach einem der vorhergehenden Ansprüche, dadurch gekennzeichnet, daß das genannte hydrophile organische Makromolekül tierischen, pflanzlichen oder marinen Ursprungs ist oder von einer Fermentation stammt, und vorzugsweise ausgewählt wird aus Keratin, Soja, Kollagen, Retikulin, Elastin, Lactalbumin, Lactoglobulin, Casein, Ovalbumin; Hyaluronsäure, Chondroitin-4-sulfat, Chondroitin-6-sulfat, Dermatansulfat, Heparansulfat, Keratansulfat sowie Heparin und seinen Derivaten oder Fraktionen mit einer Molmasse von mehr als 5000 Dalton; und Weizenproteinen, Weizen-, Mais-, Hafer-, Soja-, Mandelkeimen, Fisch-, Mollusken-, Crustaceae-, Algenproteinen, Amylose, Amylopektin, Glucomannanen, Galactomannanen, Fructosanen, Gummen, Cellulosen und Derivaten, Alginaten, Agar, Carrageenan und DNS-, RNS- Nucleinsäuren; Xanthan, Gellan, Curdlan, Dextran.

12. Verwendung nach einem der vorhergehenden Ansprüche, dadurch gekennzeichnet, daß das genannte hydrophile organische Makromolekül primäre Amin-Gruppen, wie Proteine oder Ribonucleinsäuren (DNS, RNS), umfaßt.

13. Verwendung nach einem der vorhergehenden Ansprüche, dadurch gekennzeichnet, daß das Derivat von Undecylensäure ausgewählt wird aus der Gruppe bestehend aus dem Reaktionsproduct von Undecylensäure mit einem Weizenprotein mit hoher Molmasse, einem Spirulina-Extrakt, aus Fischhaut extrahiertem Kollagen, Xanthan, DNS mit hoher Molmasse, Sojaproteinen mit hoher Molmasse, Keratin, aus Rotalgen extrahierten Proteinen mit einer Molmasse von zumindest 5000 Dalton, einer Mischung von DNS mit hoher Molmasse mit einer Fettsäure, insbesondere Laurinsäure.

14. Verwendung nach einem der vorhergehenden Ansprüche, dadurch gekennzeichnet, daß das Reaktionsprodukt einer Neutralisation durch eine organische oder mineralische Base, wie ein Amin oder ein Zink- oder Aluminiumhydroxid, unterworfen wurde.

15. Verwendung nach einem der vorhergehenden Ansprüche, dadurch gekennzeichnet, daß das Derivat von Undecylensäure in Form des Reaktionsmediums vorliegt, das durch eine organische oder mineralische Base neutralisiert wurde, und anschließend einem Lyophilisationsschritt unterworfen wurde.

16. Verwendung nach einem der vorhergehenden Ansprüche, dadurch gekennzeichnet, daß das Derivat von Undecylensäure erhalten wird, indem Undecylensäure in Mischung mit einer weiteren gesättigten oder ungesättigten Fettsäure, die insbesondere 8 bis 28 Kohlenstoffatome aufweist, umgesetzt wird.

## Claims

1. Use of an undecylenic acid derivative in the form of the product resulting from the reaction, in an aqueous medium, at room temperature, of undecylenic acid with at least one hydrophilic organic macromolecule possessing primary alcohol and/or primary amine groups and having a molecular weight greater than 2000 Daltons, as an antimicrobial, antibacterial or antifungal agent.

2. Use of an undecylenic acid derivative in the form of the product resulting from the reaction, in an aqueous medium, at room temperature, of undecylenic acid with at least one hydrophilic organic macromolecule possessing primary alcohol and/or primary amine groups and having a molecular weight greater than 2000 Daltons, as an active agent in the manufacture of a cosmetic or pharmaceutical composition with antimicrobial, antibacterial or antifungal activity, particularly in a proportion by weight of said active agent of between 0.1 and 5% by weight, based on the total weight of the composition.

3. Use according to Claim 1 or 2, characterized in that the undecylenic acid derivative is a principal or auxiliary preservative in a cosmetic or pharmaceutical composition, particularly in a proportion of between 0.1 and 5% by weight, preferably of between about 1 and about 3% by weight.

4. Use according to Claim 1 or 2, characterized in that the undecylenic acid derivative is an antidandruff agent, particularly in a proportion of between 0.1 and 5% by weight.

5. Use according to Claim 1 or 2, characterized in that the undecylenic acid derivative is an antiacne agent, particularly in a proportion of between 0.1 and 5% by weight.

6. Use according to Claim 1 or 2, characterized in that the undecylenic acid derivative is a deodorant and antiperspirant, particularly in a proportion of between 0. 1 and 5% by weight.

7. Use according to one of the preceding claims, characterized in that the proportion of undecylenic acid relative to the above-mentioned hydrophilic organic macromolecule is 10 to 75% by weight, preferably between 50 and 75% by weight.

8. Use according to one of the preceding claims, characterized in that the above-mentioned hydrophilic organic macromolecule has a molecular weight greater than 5000 Daltons, preferably of between 5000 Daltons and 1,000,000 Daltons and particularly preferably of between 50,000 Daltons and 150,000 Daltons.

9. Use according to one of the preceding claims, characterized in that the reaction product has undergone a lyophilization step.

10. Use according to one of the preceding claims, characterized in that the undecylenic acid is reacted in a reactive form, for example in the form of a halide or in the form of the anhydride.

11. Use according to one of the preceding claims, characterized in that the above-mentioned hydrophilic organic macromolecule is of animal, vegetable or marine origin or originates from fermentation and is preferably selected from keratin, silk, collagen, reticulin, elastin, lactalbumin, lactoglobulin, casein and ovalbumin; hyaluronic acid, chondroitin-4-sulphate, chondroitin-6-sulphate, dermatan sulphate, heparan sulphate and keratan sulphate, as well as heparin and its derivatives or fractions with molecular weights greater than 5000 Daltons; wheat, wheatgerm, maize, oat, soya and almond proteins, fish, mollusc, crustacean and algae proteins, amylose, amylopectin, glucomannans, galactomannans, fructosans, gums, cellulose and derivatives, alginates, aguar, carrageenan and nucleic acids (DNA, RNA); and xanthan, gellan, curdlan and dextran.

12. Use according to one of the preceding claims, characterized in that the above-mentioned hydrophilic organic macromolecule comprises primary amine groups, like proteins or ribonucleic acids (DNA, RNA).

13. Use according to one of the preceding claims, characterized in that the undecylenic acid derivative is selected from the group consisting of the reaction product of undecylenic acid and a high molecular wheat protein, spiruline extract, collagen extracted from fish skin, xanthan, high molecular DNA, high molecular silk proteins, keratin, proteins of at least 5000 Daltons extracted from pink algae, and a mixture of high molecular DNA with a fatty acid, particularly lauric acid.

14. Use according to one of the preceding claims, characterized in that the reaction product has been neutralized with an organic or mineral base such as an amine or zinc or aluminium hydroxide.

15. Use according to one of the preceding claims, characterized in that the undecylenic acid derivative is in the form of the reaction medium which has been neutralized with an organic or mineral base and then subjected to a lyophilization step.

16. Use according to one of the preceding claims, characterized in that the undecylenic acid derivative is obtained by reacting the undecylenic acid as a mixture with another saturated or unsaturated fatty acid having in particular from 8 to 28 carbon atoms.